# EUROPEAN PATENT SPECIFICATION

(11) **EP 2 972 283 B1**
(45) Date of publication and mention of the grant of the patent: **17.10.2018**
(21) Application number: 14779447.3
(22) Date of filing: 11.03.2014
(51) Int. Cl.: G01N 27/49, G01N 27/12, G01N 33/00, F23N 5/00, F23N 5/24

(54) **IMPROVED DIFFUSER DIAGNOSTIC FOR IN-SITU FLUE GAS MEASUREMENT DEVICE**
VERBESSERTE DIFFUSORDIAGNOSTIK FÜR IN-SITU-RAUCHGASMESSVORRICHTUNG
DIAGNOSTIC DE DIFFUSEUR AMÉLIORÉ POUR DISPOSITIF DE MESURE DE GAZ D'ÉVACUATION IN-SITU

(30) Priority: 13.03.2013 US 201313799416
(43) Date of publication of application: 20.01.2016
(73) Proprietor: Rosemount Analytical Inc., Solon, OH 44139 (US)
(72) Inventor: NEMER, Joseph, C., Mayfield Heights, OH 44124 (US); KRAMER, James, D., Homerville, OH 44235 (US); WEY, Anni, S., Strongsville, OH 44136 (US); SIMMERS, Douglas, E., Massillon, OH 44646 (US); SCHNEIDER, Mark, W., Orange Village, OH 44122 (US)
(74) Representative: Parker, Andrew James
(86) International application number: PCT/US2014/023459
(87) International publication number: WO 2014/164778

(56) References cited:
- US-A- 4 112 737
- US-A- 5 010 776
- US-A1- 2004 182 133
- US-A1- 2012 125 840
- US-B2- 7 927 883
- US-B2- 8 186 211

## Description

### BACKGROUND

Industrial process industries often rely on energy sources that include one or more combustion processes. Such combustion processes include operation of a furnace or boiler to generate steam or to heat a feedstock liquid. While combustion provides relatively low-cost energy, combustion efficiency is sought to be maximized, and the resulting flue gasses exiting the smokestack are often regulated. Accordingly, one goal of the combustion process management industry is to maximizing combustion efficiency of existing furnaces and boilers, which inherently also reduces the production of greenhouse gases. Combustion efficiency can be optimized by maintaining the ideal level of oxygen in the exhaust or flue gases coming from such combustion processes.

In-situ or in-process analyzers are commonly used for the monitoring, optimization, and control of the combustion processes. Typically, these analyzers employ sensors that are heated to relatively high temperatures and are operated directly above, or near, the furnace or boiler combustion zone. Known process combustion oxygen analyzers typically employ a zirconium oxide sensor disposed at an end of a probe that is inserted directly into a flue gas stream. As the exhaust or flue gas flows into the sensor, it diffuses through a filter called a diffuser into proximity with the sensor. There are no pumps or other flow-inducing devices to direct a sample flow into the sensor; the gases diffuse passively through the diffuser filter. The sensor provides an electrical signal related to the amount of oxygen present in the gas. While the diffuser allows diffusion therethrough, it also protects the sensor from physical contact with airborne solids or particulates.

Some combustion applications can adversely affect the combustion analyzer. For example, combustion processes that generate a heavy particulate load in the flue gas stream can clog or otherwise reduce the efficacy of the diffuser. When a diffuser in an in-situ probe becomes plugged, either completely or partially, the response of the analyzer to process variable changes can be slowed due to reduced or ineffective diffusion from the process to the measuring cell. Moreover, calibration errors can be caused due to back pressure on the measuring cell during calibration. Finally, at the end of a calibration cycle, the process combustion gas measurement (such as oxygen level) may still be influenced by the calibration gas. Properly detecting a plugged diffuser in a combustion process gas analyzer would reduce the possibility and effects of the problems set forth above. Moreover, given that replacement of a diffuser of a combustion process gas analyzer may require the combustion process to be taken offline, it is also not desirable to replace the diffuser unless warranted. Providing an in-situ process combustion process gas analyzer and method that are able to effectively determine when diffuser replacement or reconditioning is warranted would represent an advance in the art of combustion process monitoring.

US2004/182133 discloses a known combustion gas analyzer comprising a sensor cell assembly, and a transmitter having electrical circuitry.

### SUMMARY

A process gas analysis system according to claim 1 and a method according to claim 11 for determining a condition of the diffuser in the process gas analysis system is disclosed. The system includes a probe insertable into a source of process gas and having a distal end and a chamber proximate the distal end. A gas sensor is mounted within the chamber and is configured to provide an electrical indication relative to a species of gas. A diffuser is mounted proximate the distal end of the probe and is configured to allow gas diffusion into the chamber. A source of calibration gas is operably coupled to the probe and is configured to supply calibration gas, having a known concentration of the gas species. Electronics are coupled to the sensor and configured to store a pre-calibration process gas concentration and to measure an amount of time (sensor return time) for the sensor response to return to the pre-calibration process gas concentration. The electronics are configured to compare a measured sensor return time with a known-good sensor return time to provide an indication relative to the diffuser.

### BRIEF DESCRIPTION OF THE DRAWINGS

FIG. 1 is a diagrammatic view of an in-situ process oxygen analyzer/transmitter with which embodiments of the present invention are particularly applicable.
FIG. 2 is a diagrammatic perspective view of a combustion oxygen transmitter with which embodiments of the present invention are particularly applicable.
FIG. 3 in a diagrammatic view of a distal end of a probe disposed within a stack and measuring flue gas.
FIG. 4 is a diagrammatic view of calibration of process combustion gas sensor.
FIG. 5 is a diagrammatic view of a method of obtaining known-good process return time.
FIG. 6 is a diagrammatic view of a method of diagnosing diffuser operation in accordance with the embodiment of the present invention.

### DETAILED DESCRIPTION OF ILLUSTRATIVE EMBODIMENTS

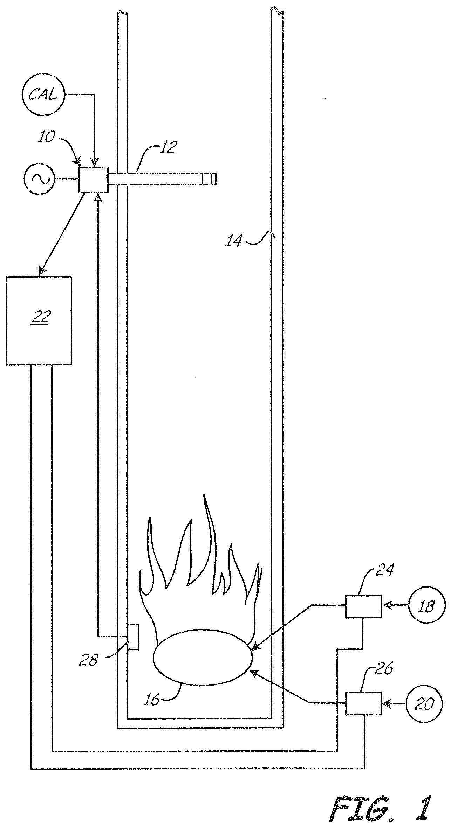

FIG. 1 is a diagrammatic view of an in-situ process oxygen analyzer/transmitter with which embodiments of the present invention are particularly applicable. Transmitter 10 can be, for example, a Model 6888 Oxygen Transmitter available from Rosemount Analytical Inc., of Solon Ohio (an Emerson Process Management Company). Transmitter 10 includes probe assembly 12 that is substantially disposed within stack or flue 14 and measures oxygen content of the flue gas related to combustion occurring at burner 16. Burner 16 is operably coupled to a source of air or oxygen 18 and source 20 of combustible fuel. Each of sources 18 and 20 is controllably coupled to burner 16 in order to control the combustion process. Transmitter 10 measures the amount of oxygen in the combustion exhaust flow and provides an indication of the oxygen level to combustion controller 22. Controller 22 controls one or both of valves 24, 26 to provide closed-loop combustion control.

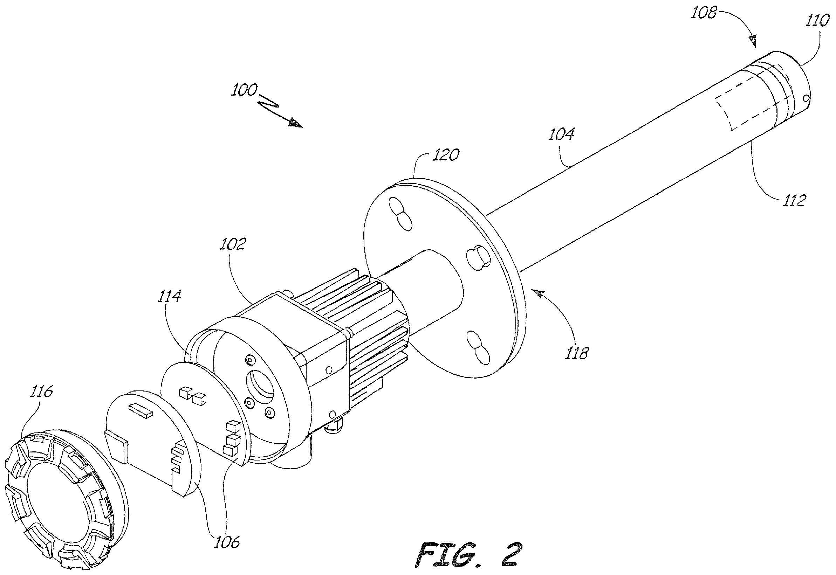

FIG. 2 is a diagrammatic perspective view of a combustion oxygen transmitter with which embodiments of the present invention are particularly applicable. Transmitter 100 includes housing 102, probe 104, and electronics 106. Probe 104 has a distal end 108 where a diffuser 110 is mounted. The diffuser is a physical device that allows at least some gaseous diffusion therethrough, but otherwise protects components within probe 104. Specifically, diffuser 110 protects a measurement cell, or sensor 112, illustrated in phantom in FIG. 2.

Housing 102 has a chamber 114 that is sized to house electronics 106. Additionally, housing 102 includes internal threads that are adapted to receive and mate with external threads of end cap 116 to form a hermetic seal. Additionally, housing 102 includes a bore or aperture therethrough allowing electrical interconnection between electronics 106 and measuring cell or sensor 112 disposed within distal end 108 of probe 104.

Probe 104 is configured to extend within a flue, such as flue 14. Probe 104 includes a proximal end 118 that is adjacent flange 120. Flange 120 is used to mount or otherwise secure the transmitter 100 to the sidewall of the flue. When so mounted, transmitter 100 may be completely supported by the coupling of flange 120 to the flue wall.

Electronics 106 provide heater control and signal conditioning, resulting in a linear 4-20 mA signal representing flue gas oxygen. Preferably, electronics 106 also includes a microprocessor that is able to execute programmatic steps to provide the functions of diffuser diagnostics as will set forth in greater detail below. However, in some embodiments, transmitter 100 may simply be "a direct replacement" probe with no electronics and thus sending raw millivolt signals for the sensing cell and thermocouple providing indications representative of the oxygen concentration and cell temperature, respectively. In embodiments where a "direct replacement" probe is used, the probe is coupled to a suitable analyzer such as the known Xi Operator Interface available from Rosemount Analytical Inc. The Xi Operator Interface provides a back-lit display, signal conditioning and heater control within a NEMA 4X (IP 66) enclosure. The electronics of the Xi Operator Interface also provides features, such as automatic calibration, stoichiometer indications in reducing conditions, and programmable reference features for measuring at near-ambient levels. Accordingly, the Xi Operator Interface includes suitable processing abilities to perform diffuser diagnostics in accordance with embodiments of the present invention. Thus, in applications where the transmitter is a "direct replacement" probe embodiments of the present inventions can still be practiced.

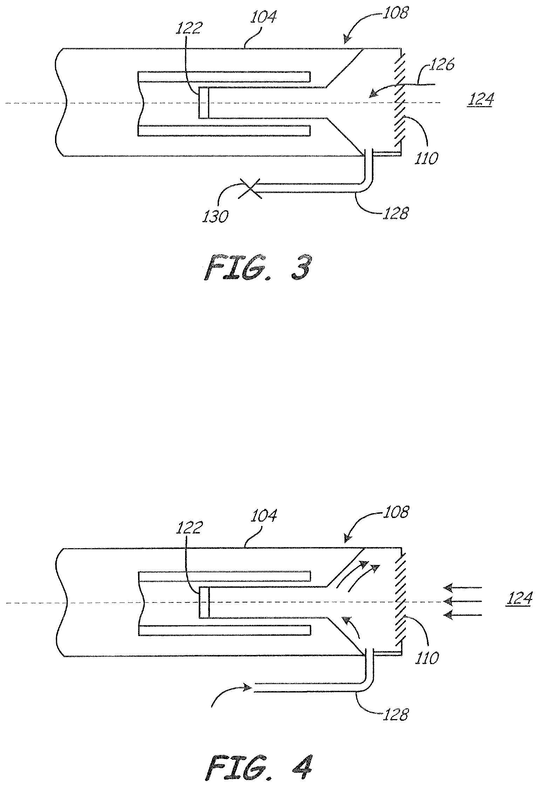

Over time, it is periodically necessary to calibrate sensor 122. Embodiments of the present invention generally leverage the behavior of the oxygen sensor occurring between a calibration mode and a process monitoring mode. For reference, both modes are described with respect to FIGS. 3 and 4, below.

FIG. 3 in a diagrammatic view of distal end 108 of probe 104 disposed within a stack and measuring flue gas 124 during a process monitoring mode. Flue gas 124 diffuses through diffuser 110 as illustrated at reference numeral 126. A calibration line 128 is closed or otherwise obstructed as indicated at reference numeral 130. During monitoring of combustion gas 124, such gas diffuses through diffuser 110 and contacts sensor 122. Sensor 122 is electrically coupled to suitable electronics, such as electronics 106, or an external analyzer such as the Xi Operator Interface described above. Sensor 122 generates a signal that is indicative of the oxygen concentration of gas contacting sensor 122, and is thus indicative of oxygen present within flue gas 124. As can be appreciated, if diffuser 110 becomes blocked, either partially or fully, the ability of sensor 122 to accurately measure oxygen of flue gas 124 is compromised.

FIG. 4 is a diagrammatic view of calibration of sensor 122. During calibration, calibration line 128 is operably coupled to a source of calibration gas. Calibration gas is any gas that has a known oxygen content. The calibration gas flows into distal end 108 of probe 104 between sensor 122 and diffuser 110. Sufficient calibration gas is flowed until the entire chamber within distal end 108 is filled with the calibration gas. At such time, sensor 122 will reflect a value that is indicative of its reading of the oxygen content of the calibration gas. Given that the calibration gas has a known oxygen content, any errors, drift, or other inaccuracies of sensor 122 can be measured and removed.

According to the invention the temporal response of the oxygen sensor between the calibration mode and the process monitoring mode is measured. The temporal response of the oxygen sensor can be analyzed to detect when diffuser 110 is plugged, either completely or partially. When the oxygen transmitter is new, either just manufactured, or newly installed, a sensor return time value is obtained for a known good configuration. For example, the analyzer can be installed into a new combustion installation, and can be operated to read a flue gas oxygen concentration. In accordance with the present invention, just prior to calibration, the flue gas oxygen concentration is stored in memory, either the memory of electronics of the oxygen transmitter, or memory of the external device that is coupled to the direct replacement probe. Then, calibration is initiated wherein a calibration gas having a known oxygen concentration is flowed into the distal end of the probe between the measuring sensor and the diffuser. The calibration gas is flowed for a suitable length of time to ensure that all combustion gas is removed from the distal end. Then, a measurement of the calibration gas oxygen content is obtained from the sensor. A suitable amount of time can be a specific time, such as one minute, or can be based upon the sensor response, such that when the sensor response change level is below a certain threshold (indicating substantial steady state) then the calibration measurement can be made. Once the calibration measurement has been made, the calibration gas flow is ceased, a timer is initiated and the sensor output is monitored. The timer is used to measure the length of the time from the cessation of the calibration gas to the point in time where the sensor measures a combustion gas oxygen amount that matches the value that was stored just prior to calibration. Since, the measured time is obtained during a known good configuration, it is stored as a known-good sensor return time or threshold. Alternatively, the known-good threshold can simply be programmed into the transmitter at the time of manufacture. Further still, the method may wait until the sensor is indicating substantial steady state. The objective is to have confidence that the sensor has returned to the combustion gas measurement, which may have changed during calibration.

Later, after the transmitter has operated for some time, such as months or years, each time a calibration cycle is performed, the time required for the combustion gas sensor to return to the process oxygen value, stored just prior to a calibration, is compared with the known-good configuration threshold. This comparison may be a simple comparison to determine if the later time measurement is equal to or less than the known-good threshold, thus indicating that the diffuser is operating effectively. Additionally, a small buffer can be added to the known-good time threshold such that a slight amount of obstruction can be tolerated. For example, the measured sensor return time can be compared to the known-good threshold and if the measured sensor return time is at or below 110% of the known-good threshold, the diffuser can be indicated as being effective. Conversely, if the measured sensor return time exceeds the known-good threshold with the optional buffer, then an indication can be provided that the diffuser has deteriorated to such an extent that it requires replacement or repair. This alert can be provided through a process communication loop, either using a known process communication protocol, such as the digital Highway Addressable Remote Transducer (HART®) communication standard, through a local operator interface, or both depending upon the application. Additionally, a local enunciator, such as an LCD or an audible alarm can be provided at the transmitter itself.

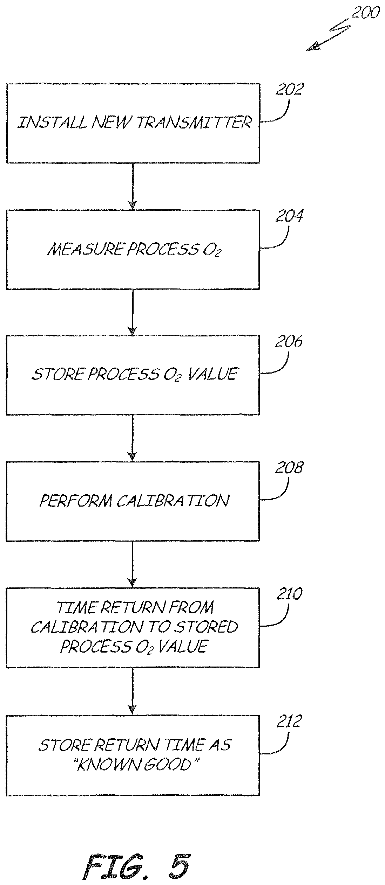

FIG. 5 is a diagrammatic view of a method of obtaining known-good process return time. Method 200 begins at block 202 where a new transmitter is installed in a process installation. Next, at block 204, the transmitter is operated to measure a combustion process oxygen level. The measured combustion process oxygen level is stored, as indicated at block 206. Method 200 continues, at block 208, with a calibration of the transmitter. Immediately after calibration 208, calibration gas flow is ceased and block 210 executes to begin timing the amount of time required for the oxygen sensor value to return from the calibration value to a value equal to the stored process oxygen value. The amount of time measured in block 210 is then stored within memory of the electronics, such as electronics 106 of the transmitter, or electronics of a suitable external device, such as the Xi Operator Interface. The stored known-good return time is used subsequently to compare against subsequently measured sensor return times to determine diffuser obstruction in accordance with embodiments of the present invention.

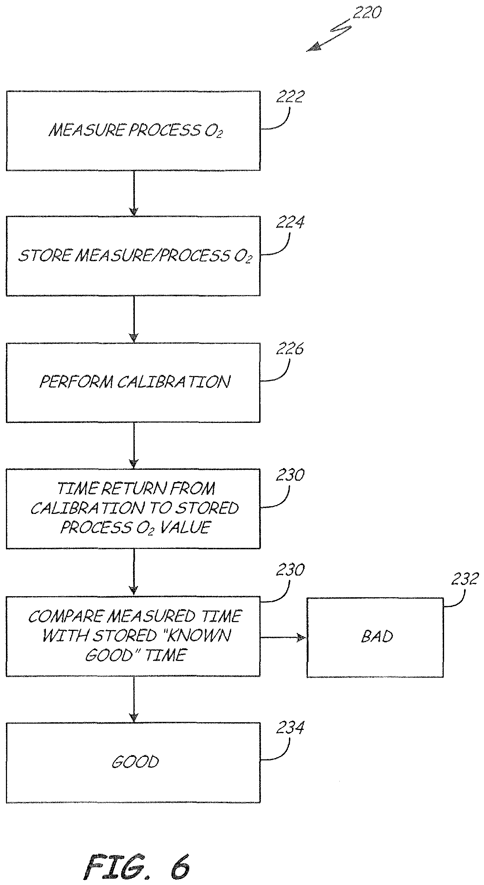

FIG. 6 is a diagrammatic view of a method of diagnosing diffuser operation in accordance with the present invention. Method 220 begins at block 222 where the transmitter is used to measure a process oxygen level. Next, at block 224, the measured process oxygen level is stored within memory of suitable electronics, such as electronics 106 of the oxygen transmitter itself, or electronics of a suitable external analyzer. At block 226, calibration of the transmitter is performed. Next, at block 228, as the calibration gas flow is ceased, a timer is initiated to measure the amount of time for the sensor reading to return from the calibration value to a value equal to the stored process oxygen value or to a substantial steady state of the process oxygen value. Next, at block 230, the measured sensor return time from block 228 is compared to the stored known-good return time as obtained at block 212 (described with respect to FIG. 5). As a result of this comparison, a processor, such as the processor of electronics 106, or a suitable external analyzer, provides an indication relative to the diffuser. Specifically, if the measured return time exceeds the known-good return time either exactly, or exceeds the known-good time by a specified buffer, the diffuser is indicated as requiring repair or replacement, as indicated at block 232. Conversely, if the measured return time is less than or equal to the known-good return time or is less than the known-good return time added to a specified buffer, the diffuser is indicated as good at block 234.

Embodiments of the present invention generally provide a method that is easily implemented in existing hardware to allow processors, such as the processor of the transmitter, or a processor of an operator interface to provide a diagnostic indication relative to the diffuser of the transmitter. This allows a technician to be alerted precisely when diffuser replacement or repair is required. Thus, accurate and timely measurements of combustion oxygen are provided, and technician time required to replace or refurbish the diffuser is minimized.

## Claims

1. A process gas analysis system comprising:
a probe (104) insertable into a source of process gas, the probe (104) having a distal end and a chamber (114) proximate the distal end (108);
a gas sensor (122), which is an Oxygen sensor, configured to provide an electrical indication relative to a species of gas, the gas sensor (122) being mounted within the chamber (114);
a diffuser (110) mounted proximate the distal end (108) of the probe (104), the diffuser (110) being configured to allow gas diffusion into the chamber;
a source of calibration gas operably coupled to the probe (104), the source of calibration gas being configured to supply calibration gas, having a known concentration of the gas species;
electronics (106) coupled to the sensor (122) and configured to provide a process monitoring mode that senses the species of gas using the gas sensor (122), and a calibration mode where the gas sensor (122) senses the calibration gas and wherein the electronics (106) are configured to store a pre-calibration process gas concentration, and to measure a sensor return time, wherein the sensor return time comprises an amount of time required for the sensor response to return to the pre-calibration process gas concentration after calibration mode, before process monitoring mode; and
wherein the electronics (106) are configured to compare the measured sensor return time with a stored known-good sensor return time and provide an indication whether or not the diffuser (110) requires repair or replacement.

2. The process gas analysis system of claim 1, wherein the electronics (106) are a component of a process gas transmitter (100).

3. The process gas analysis system of any of the preceding claims, wherein the electronics (106) are a component of an operator interface coupled to the probe (104).

4. The process gas analysis system of any of the preceding claims, wherein the known-good sensor return time is obtained when the system is first operated.

5. The process gas analysis system of any of the preceding claims, wherein the electronics (106) are configured to provide a diagnostic indication relative to the diffuser (110) if the measured sensor return time exceeds the known-good sensor return time.

6. The process gas analysis system of any of the preceding claims, wherein the electronics (106) are configured to provide a diagnostic indication relative to the diffuser (110) if the measured sensor return time exceeds the known-good sensor return time by a specified buffer.

7. The process gas analysis system of any of the preceding claims, wherein the indication notifies a technician to replace the diffuser (110).

8. The process gas analysis system of any of the preceding claims, wherein the indication is indicative of a partially obstructed diffuser (110).

9. The process gas analysis system of any of the preceding claims, wherein the indication is provided over a process communication loop.

10. The process gas analysis system of any of the preceding claims, wherein the indication is provided locally.

11. A method of determining a condition of the diffuser in the process gas analysis system according to any one of the previous claims, the gas analysis system having a process monitoring mode and calibration mode, the method comprising:
storing a pre-calibration process gas concentration value;
performing a calibration on the process gas analysis system;
measuring an amount of time required for the sensor (122) of the system to return to the stored pre-calibration value after calibration mode, but before process monitoring mode;
generating a comparison between the measured amount of time and a stored, known-good sensor return time; and
providing a diffuser diagnostic indication based on the comparison, wherein the diffuser diagnostic indication at least comprises a determination of whether or not the diffuser (110) requires repair or replacement.

12. The method of any of the preceding claims, wherein the stored, known-good sensor return time is stored during manufacture of the system.

13. The method of any of the preceding claims, wherein the known-good sensor return time is stored after a calibration performed when the system is first installed in a process.

## Patentansprüche

1. Prozessgas-Analysesystem, das Folgendes aufweist:
eine Sonde (104), die in eine Prozessgas-Quelle einführbar ist, wobei die Sonde (104) ein distales Ende und eine an das distale Ende (108) anschließende Kammer (114) aufweist;
einen Gassensor (122), der ein Sauerstoffsensor ist und der dazu ausgebildet ist, eine elektrische Angabe hinsichtlich einer Gasart anzugeben, wobei der Gassensor (122) in der Kammer (114) montiert ist;
ein Diffusor (110), der nahe dem distalen Ende (108) der Sonde (104) montiert ist, wobei der Diffusor (110) dazu ausgebildet ist, Gasdiffusion in die Kammer zu ermöglichen;
eine Kalibriergas-Quelle, die funktionell an die Sonde (104) gekoppelt ist, wobei die Kalibriergas-Quelle dazu ausgebildet ist, Kalibriergas bereitzustellen, das eine bekannte Konzentration der Gasart aufweist;
Elektronik (106), die an den Sensor (122) gekoppelt ist und die dazu ausgebildet ist, einen Prozess-Überwachungsmodus anzugeben, der unter Verwendung des Gassensors (122) die Gasart detektiert, und die dazu ausgebildet ist, einen Kalibrierungsmodus anzugeben, bei dem der Gassensor (122) das Kalibriergas detektiert, wobei die Elektronik (106) dazu ausgebildet ist, eine Vorkalibrierungs-Prozessgas-Konzentration zu speichern, und die dazu ausgebildet ist, eine Sensor-Rücklaufzeit zu messen, wobei die Sensor-Rücklaufzeit eine Zeitmenge angibt, die für die Sensorantwort erforderlich ist, um nach dem Kalibrierungsmodus und vor dem Prozess-Überwachungsmodus zu der Vorkalibrierungs-Prozessgas-Konzentration zurückzukehren; und
wobei die Elektronik (106) dazu ausgebildet ist, die gemessene Sensor-Rücklaufzeit mit einer gespeicherten, bekannt guten Sensor-Rücklaufzeit zu vergleichen und eine Angabe anzugeben, ob eine Reparatur oder ein Ersetzen des Diffusors (110) erforderlich ist oder nicht.

2. Prozessgas-Analysesystem gemäß Anspruch 1, wobei die Elektronik (106) eine Komponente eines Prozessgas-Transmitters (100) ist.

3. Prozessgas-Analysesystem gemäß einem der vorhergehenden Ansprüche, wobei die Elektronik (106) eine Komponente einer Bedienoberfläche ist, die an die Sonde (104) gekoppelt ist.

4. Prozessgas-Analysesystem gemäß einem der vorhergehenden Ansprüche, wobei die bekannt gute Sensor-Rücklaufzeit erhalten wird, wenn das System zum ersten Mal in Betrieb genommen wird.

5. Prozessgas-Analysesystem gemäß einem der vorhergehenden Ansprüche, wobei die Elektronik (106) dazu ausgebildet ist, eine diagnostische Angabe hinsichtlich des Diffusors (110) anzugeben, sofern die gemessene Sensor-Rücklaufzeit die bekannt gute Sensor-Rücklaufzeit überschreitet.

6. Prozessgas-Analysesystem gemäß einem der vorhergehenden Ansprüche, wobei die Elektronik (106) dazu ausgebildet ist, eine diagnostische Angabe hinsichtlich des Diffusors (110) anzugeben, sofern die gemessene Sensor-Rücklaufzeit die bekannt gute Sensor-Rücklaufzeit um einen bestimmten Puffer überschreitet.

7. Prozessgas-Analysesystem gemäß einem der vorhergehenden Ansprüche, wobei die Angabe einen Techniker benachrichtigt, den Diffusor (110) zu ersetzen.

8. Prozessgas-Analysesystem gemäß einem der vorhergehenden Ansprüche, wobei die Angabe für einen teilweise blockierten/verstopften Diffusor (110) indikativ ist.

9. Prozessgas-Analysesystem gemäß einem der vorhergehenden Ansprüche, wobei die Angabe über eine Prozess-Kommunikationsschleife angegeben wird.

10. Prozessgas-Analysesystem gemäß einem der vorhergehenden Ansprüche, wobei die Angabe lokal angegeben wird.

11. Ein Verfahren zum Bestimmen eines Zustands des Diffusors in dem Prozessgas-Analysesystem gemäß einem der vorhergehenden Ansprüche, wobei das Gas-Analysesystem einen Prozess-Überwachungsmodus und einen Kalibrierungsmodus aufweist und das Verfahren folgende Schritte aufweist:
Speichern eines Vorkalibrierungs-Prozessgas-Konzentrationswertes;
Durchführen einer Kalibrierung des Prozessgas-Analysesystems;
Messen einer Zeitmenge, die für den Sensor (122) des Systems erforderlich ist, um den gespeicherten Vorkalibrierungs-Wert nach dem Kalibrierungsmodus und vor dem Prozess-Überwachungsmodus zurückzuführen;
Erzeugen eines Vergleichs zwischen der gemessenen Zeitmenge und einer gespeicherten, bekannt guten Sensor-Rücklaufzeit; und
Angeben einer Diffusor-Diagnoseangabe basierend auf dem Vergleich, wobei die Diffusor-Diagnoseangabe zumindest eine Feststellung aufweist, ob eine Reparatur oder ein Ersetzen des Diffusors (110) erforderlich ist oder nicht.

12. Verfahren gemäß einem der vorhergehenden Ansprüche, wobei die gespeicherte, bekannt gute Sensor-Rücklaufzeit während der Herstellung des Systems gespeichert wird.

13. Verfahren gemäß einem der vorhergehenden Ansprüche, wobei die bekannt gute Sensor-Rücklaufzeit nach einer Kalibrierung gespeichert wird, die durchgeführt wird, wenn das System zum ersten Mal in einem Prozess installiert wird.

## Revendications

1. Système d'analyse pour gaz de traitement comprenant :
une sonde (104) susceptible d'être insérée dans une source de gaz de traitement, la sonde (104) ayant une extrémité distale et une chambre (114) à proximité de l'extrémité distale (108) ;
un détecteur de gaz (122), qui est un détecteur d'oxygène, configuré pour donner une indication électrique en relation avec une espèce de gaz, le détecteur de gaz (122) étant monté à l'intérieur de la chambre (114) ;
un diffuseur (110) monté à proximité de l'extrémité distale (108) de la sonde (104), le diffuseur (110) étant configuré pour permettre la diffusion d'un gaz entrant dans la chambre ;
une source de gaz de calibrage fonctionnellement couplée à la sonde (104), la source de gaz de calibrage étant configurée pour alimenter un gaz de calibrage, ayant une concentration connue de l'espèce de gaz ;
un système électronique (106) couplé au détecteur (122) et configuré pour assurer un mode de surveillance de traitement qui détecte l'espèce de gaz en utilisant le détecteur de gaz (122), et un mode de calibrage dans lequel le détecteur de gaz (122) détecte le gaz de calibrage, et dans lequel le système électronique (106) est configuré pour stocker une concentration de gaz de traitement de précalibrage, et pour mesurer un temps de retour du détecteur, tel que le temps de retour du détecteur comprend une durée temporelle requise pour que la réponse du détecteur retourne à la concentration de gaz de traitement de précalibrage après le mode de calibrage et avant le mode de surveillance de traitement ; et
dans lequel le système électronique (106) est configuré pour comparer le temps de retour mesuré du détecteur avec un temps de retour stocké reconnu valable du détecteur et pour donner une indication quant à savoir si le diffuseur (110) a besoin ou non d'une réparation ou d'un remplacement.

2. Système d'analyse pour gaz de traitement selon la revendication 1, dans lequel le système électronique (106) est un composant d'un transmetteur de gaz de traitement (100).

3. Système d'analyse pour gaz de traitement selon l'une quelconque des revendications précédentes, dans lequel le système électronique (106) est un composant d'une interface opérateur couplée à la sonde (104).

4. Système d'analyse pour gaz de traitement selon l'une quelconque des revendications précédentes, dans lequel le temps de retour reconnu valable du détecteur est obtenu lorsque le système est mis en fonctionnement en premier.

5. Système d'analyse pour gaz de traitement selon l'une quelconque des revendications précédentes, dans lequel le système électronique (106) est configuré pour donner une indication de diagnostic par rapport au diffuseur (110) si le temps de retour mesuré du détecteur excède le temps de retour reconnu valable du détecteur.

6. Système d'analyse pour gaz de traitement selon l'une quelconque des revendications précédentes, dans lequel le système électronique (106) est configuré pour donner une indication de diagnostic par rapport au diffuseur (110) si le temps de retour mesuré du détecteur excède le temps de retour reconnu valable du détecteur à raison d'une durée tampon spécifiée.

7. Système d'analyse pour gaz de traitement selon l'une quelconque des revendications précédentes, dans lequel l'indication notifie à un technicien de remplacer le diffuseur (110).

8. Système d'analyse pour gaz de traitement selon l'une quelconque des revendications précédentes, dans lequel l'indication indique un diffuseur partiellement obstrué (110).

9. Système d'analyse pour gaz de traitement selon l'une quelconque des revendications précédentes, dans lequel l'indication est fournie via une boucle de communication de traitement.

10. Système d'analyse pour gaz de traitement selon l'une quelconque des revendications précédentes, dans lequel l'indication est donnée localement.

11. Procédé pour déterminer une condition du diffuseur dans le système d'analyse pour gaz de traitement selon l'une quelconque des revendications précédentes, le système d'analyse pour gaz ayant un mode de surveillance de traitement et un mode de calibrage, le procédé comprenant les étapes consistant à :
stocker une valeur de concentration de gaz de traitement de précalibrage ;
effectuer un calibrage sur le système d'analyse pour gaz de traitement ;
mesurer une durée temporelle requise pour que le détecteur (122) du système retourne à la valeur de précalibrage stockée après le mode de calibrage, mais avant le mode de surveillance de traitement ;
générer une comparaison entre la durée temporelle mesurée et un temps de retour stocké reconnu valable du détecteur ; et
fournir une indication de diagnostic du diffuseur sur la base de la comparaison, de sorte que l'indication de diagnostic du diffuseur comprend au moins une détermination quant à savoir si le diffuseur (110) nécessite ou non une réparation ou un remplacement.

12. Procédé selon l'une quelconque des revendications précédentes, dans lequel le temps de retour stocké reconnu valable du détecteur est stocké pendant la fabrication du système.

13. Procédé selon l'une quelconque des revendications précédentes, dans lequel le temps de retour reconnu pour valable du détecteur est stocké après un calibrage exécuté quand le système est d'abord installé dans un traitement.
